# EUROPEAN PATENT SPECIFICATION

(11) **EP 0 709 377 B1**
(45) Date of publication and mention of the grant of the patent: **29.09.1999**
(21) Application number: 95116957.2
(22) Date of filing: 27.10.1995
(51) Int. Cl.: C07D 215/52, C07D 215/14, C07D 401/06, C07D 471/04

(54) **Process for producing quinolin-2-yl benzoic acid compounds**
Verfahren zur Herstellung von Chinolin-2-yl-benzoesäure
Procédé pour la préparation de composés d'acide benzoique

(30) Priority: 27.10.1994 JP 26412194; 27.10.1994 JP 26412294
(43) Date of publication of application: 01.05.1996
(73) Proprietor: ASAHI GLASS COMPANY LTD., Chiyoda-ku, Tokyo (JP); YOSHITOMI PHARMACEUTICAL INDUSTRIES, LTD., Chuo-ku, Osaka-shi (JP)
(72) Inventor: Wang, Shuzhong, c/o Asahi Glass Co., Ltd., Yokohama-shi, Kanagawa (JP); Ito, Tomoko, c/o Asahi Glass Co., Ltd., Yokohama-shi, Kanagawa (JP); Okazoe, Takashi, c/o Asahi Glass Co., Ltd., Yokohama-shi, Kanagawa (JP); Morizawa, Yoshitomi, c/o Asahi Glass Co., Ltd., Yokohama-shi, Kanagawa (JP)
(74) Representative: Hansen, Bernd, Dr. Dipl.-Chem.

(56) References cited:
- EP-A- 0 569 013

## Description

### FIELD OF THE INVENTION

The present invention relates to a process for producing quinolin-2-yl benzoic acid compounds useful as intermediates of angiotensin II antagonists which are effective for treating hypertension.

### BACKGROUND OF THE INVENTION

Blood pressure in the living body is controlled mainly by the sympathetic nerve system and the balance of pressor and depressor systems. The renin-angiotensin system is a pressor system. In the renin-angiotensin system, renin acts on angiotensinogen to form angiotensin I which is subsequently converted into angiotensin II by the action of an angiotensin converting enzyme. Angiotensin II shows strong angiotonic activity and acts on the adrenal cortex to enhance secretion of aldosterone, thus increasing the blood pressure. Since angiotensin II exerts its function through the angiotensin II receptor located on the cell membrane, its antagonist can be used, in addition to an angiotensin converting enzyme inhibitor, as a therapeutic agent for the treatment of hypertension caused by angiotensin II.

Angiotensin II antagonist peptides such as saralasin are known in the prior art. However, peptides are not effective when administered orally. Recently, non-peptide angiotensin II antagonists have been reported, for example, in JP-A-56-71074, JP-W-3-501020 and WO93/19060, and their efficacy by oral administration has been confirmed. (The terms "JP-A" and "JP-W" as used herein means an "unexamined published Japanese patent application" and "unexamined PCT application published in Japan, respectively).

The present inventors conducted extensive studies in order to find non-peptide compounds having an angiotensin II antagonism and being effective when administered orally. As a result, they successfully found that novel quinoline derivatives would satisfy these requirements (JP-A-6-16659 and JP-A-6-80664). They have further applied inventions relating to quinolin-2-yl benzoic acids, which are intermediates in the synthesis of these novel quinoline derivatives, and a process for producing the same for a patent (JP-A-6-16641). EP-A-0 569 013 belongs to the same patent family as these three Japanese applications.

The process for producing quinolin-2-yl benzoic acids according to the above-mentioned invention (JP-A-6-16641) comprises reacting an isatin with an acylbenzoic acid to thereby give a carboxyphenylquinolinecarboxylic acid, converting the resulting product into a diester, selectively hydrolyzing the ester on the quinoline ring and then decarboxylating the carboxyl group on the quinoline ring to thereby give a quinolin-2-yl benzoic acid derivative. In this process, however, it is necessary to synthesize a compound, wherein the carboxyl group on the benzene ring of the carboxyphenylquinolinecarboxylic acid is protected by esterification while the carboxyl group on the quinoline ring is not protected, followed by decarboxylation. A complicated process involving a number of steps is required for synthesizing the dicarboxylic acid derivative, wherein one carboxyl group has been esterified while another is not esterified, from a dicarboxylic acid.

### SUMMARY OF THE INVENTION

An object of the present invention is to provide a process for efficiently producing quinolin-2-yl benzoic acid compounds.

The present inventors have conducted extensive studies in order to attain the above object. As a result, they have found that the aimed quinolin-2-yl benzoic acid derivative can be synthesized by decarboxylating the above-mentioned dicarboxylic acid as such, i.e., without protecting both of the two carboxyl groups.

The present invention provides a process for producing a quinolin-2-yl benzoic acid compound characterized by decarboxylating a 2-carboxyphenyl-4-quinolinecarboxylic acid compound in which two carboxyl groups are not substituted and a benzene ring and a quinoline ring may have one or more substituents inert to said decarboxylation reaction.

### DETAILED DESCRIPTION OF THE INVENTION

The 2-carboxyphenyl-4-quinolinecarboxylic acid compound to be used in the present invention has two free carboxyl groups (-COOH). These carboxyl groups should not be denatured, for example, by esterification, and the carboxyl group on the quinoline ring should be bonded at the 4-position of the ring. On the other hand, the carboxyl group on the benzene ring may be bonded at an arbitrary position of the ring, though it is preferably bonded at the 2-position.

The quinoline ring and the benzene ring of the 2-carboxyphenyl-4-quinolinecarboxylic acid may have substituents which remain inert during the decarboxylation. Two or more substituents, which may be different from each other, may be bonded to each ring.

In the present invention, the decarboxylation takes place at the carboxyl group bonded to the quinoline ring, while the carboxyl group bonded to the benzene ring does not substantially change. Therefore, it is not necessary to esterify the carboxyl group bonded to the benzene ring for protection, as in the conventional cases.

Preferable examples of the 2-carboxyphenyl-4-quinolinecarboxylic acid to be used in the present invention include the compounds represented by the following formula (1). By decarboxylating these compounds, the corresponding quinolin-2-yl benzoic acids represented by the following formula (2) can be obtained. 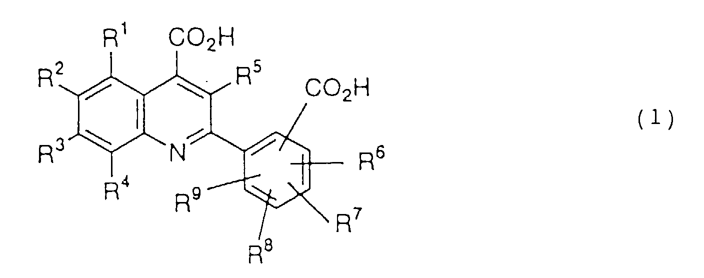 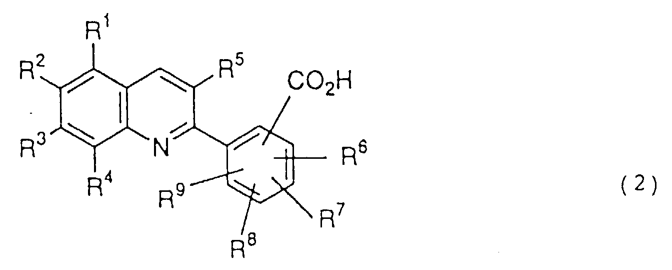 wherein R¹, R³, R⁴, R⁵, R⁶, R⁷, R⁸ and R⁹ each independently represents a hydrogen atom, a halogen atom, a lower alkyl group, a lower cycloalkyl group, an aryl group, an aralkyl group, an alkoxy group or -CₘF₂ₘ₊₁; and R² represents a hydrogen atom, a halogen atom, a lower alkyl group, a cyclo lower alkyl group, an aryl group, an aralkyl group, an alkoxy group, -CₙF₂ₙ₊₁ or -CH₂Q. Q represents a monovalent organic group derived from an organic compound having an -NH-group by eliminating the hydrogen atom bonded to said nitrogen atom, and m and n each independently represents an integer of from 1 to 6.

The term "lower" as used herein with regard to organic groups means that each group has from 1 to 6 carbon atoms. Suitable examples of the "lower alkyl group" include methyl, ethyl, propyl, isopropyl, butyl, isobutyl, t-butyl, pentyl and hexyl groups. The term "cyclo lower alkyl group" means a cycloalkyl group having from 3 to 6 carbon atoms. Suitable examples thereof include cyclopropyl, cyclobutyl, cyclopentyl and cyclohexyl groups.

The term "halogen atom" means a fluorine, chlorine, bromine or iodine atom. The term "aryl group" means a monovalent aromatic hydrocarbon group and a phenyl group and derivatives thereof are preferable. Suitable examples thereof include phenyl, tolyl and p-halophenyl groups. The term "aralkyl group" means an alkyl group substituted with an aryl group. It is preferable that the alkyl group has 1 to 4 carbon atoms. Suitable examples thereof include benzyl, benzhydryl, trityl and phenethyl groups. As the "alkoxy group", a lower alkoxy group is preferable and one having 1 to 4 carbon atoms is more preferable. Suitable examples thereof include methoxy, ethoxy, propoxy and butoxy groups.

It is preferable that R¹, R², R³, R⁴ and R⁵ each independently represents a hydrogen atom, a lower alkyl group or an aryl group, more preferably a hydrogen atom or a lower alkyl group having 1 to 4 carbon atoms. In addition, it is preferable that R² is -CH₂Q. At the same time, it is preferable that R⁶, R⁷, R⁸ and R⁹ each independently represents a hydrogen atom, a halogen atom or a lower alkyl group. As the halogen atom, a chlorine or fluorine atom is preferable.

It is preferable that R² is a hydrogen atom, a methyl group or -CH₂Q, in particular, a methyl group or -CH₂Q. More specifically, a methyl group serves as a connecting group between an imidazole ring, an imidazopyridine ring or the like and the quinoline skeleton in a quinoline angiotensin II antagonist (JP-A-6-16659 and JP-A-6-80664) and -CH₂Q itself has preferably the imidazole ring, imidazopyridine ring or the like. In such a case, it is preferable that R¹ to R⁵ excluding R² are each a hydrogen atom.

It is preferable that R⁶, R⁷, R⁸ and R⁹ are all hydrogen atoms or at least one of them is a halogen atom (in particular, a chlorine atom) while the others are hydrogen atoms.

Q represents a monovalent organic group obtained from an organic compound having an -NH-group by eliminating the hydrogen atom bonded to said nitrogen atom. This organic compound having an -NH- group is preferably a heterocyclic compound wherein the nitrogen atom in the -NH- group constitutes the ring. This heterocyclic compound may be a condensed one. The organic compound having an -NH- group may be an aliphatic amine compound, an alicyclic amine compound or an aromatic amine compound, having at least one primary or secondary amino group.

Preferable examples of Q include residues of heterocyclic compounds such as residues of substituted imidazole compound and substituted imidazopyridine compounds described in JP-A-6-16659 and JP-A-6-80664. Namely, Q is preferably a substituted 1H-imidazol-1-yl group represented by the following formula (3), more preferably a substituted 3H-imidazo[4,5-b]pyridin-3-yl group represented by the following formula (4). 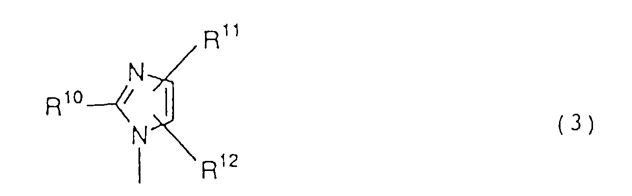 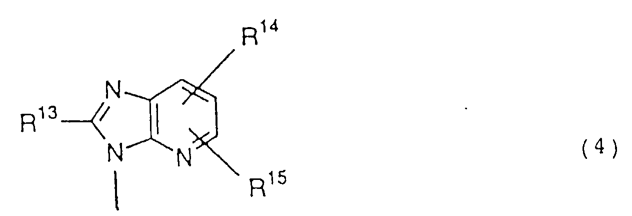 wherein
R¹⁰ and R¹³ each independently represents a lower alkyl group, a halo lower alkyl group, a cyclo lower alkyl group, an alkenyl group, an alkoxy group, a alkoxy lower alkyl group or an alkylthio group;
R¹¹ and R¹² may be the same or different and each independently represents a hydrogen atom, a halogen atom, CᵢF₂ᵢ₊₁-, -(CH₂)ₚR²⁰,-(CH₂)ᵣCOR²¹ or -(CH₂)ₜNR²²COR²³;
R¹⁴ and R¹⁵ may be the same or different and each independently represents a hydrogen atom, a halogen atom, a lower alkyl group, a halo lower alkyl group, a cyclo lower alkyl group, an alkenyl group, an alkoxy group, CⱼF₂ⱼ₊₁-, -(CH₂)_{q}R²⁴ or-(CH₂)ₛCOR²⁵;
R²⁰ and R²⁴ each independently represents a hydroxyl group or an alkoxy group;
R²¹ and R²⁵ each independently represents a hydrogen atom, a lower alkyl group or an alkoxy group;
R²² represents a hydrogen atom or a lower alkyl group;
R²³ represents a hydrogen atom, a lower alkyl group or an alkoxy group;
i and j independently represent an integer of from 1 to 6;
p and q independently represent an integer of from 1 to 4;
r and s independently represent 0 or an integer of from 1 to 4; and
t represents 0 or an integer of from 1 to 4.

Preferably, R¹⁰ and R¹³ are each a lower alkyl group independently; R¹¹ is a chlorine atom; R¹² is -(CH₂)ₚR²⁰, wherein R²⁰ is a hydroxyl group and p is 1, or -(CH₂)ᵣCOR²¹, wherein R²¹ is a hydrogen atom or a lower alkoxy group and r is 0 or 1; R¹⁴ and R¹⁵ may be either the same or different and each independently represents a hydrogen atom, a lower alkyl group, -(CH₂)_{q}R²⁴, wherein R²⁴ is a hydroxyl group and q is 1, or -(CH₂)ₛCOR²⁵, wherein R²⁵ is a hydrogen atom or a lower alkoxy group and s is 0 or 1.

Since the target compounds of the present invention, i.e., the quinolin-2-yl benzoic acid compounds are particularly useful as intermediates for producing the above-mentioned quinoline angiotensin II antagonists, compounds having a carboxyl group at the 2-position are preferable. However, the present invention is not restricted thereto and those having a carboxyl group at any other position are also usable. The most desirable quinolin-2-yl benzoic acid compounds of the present invention are 2-(6-methylquinolin-2-yl)benzoic acid and 2-(6-substituted quinolin-2-yl)benzoic acids having -CH₂Q at the 6-position of quinoline wherein Q is a substituted 1H-imdazol-1-yl group or a substituted 3H-imidazo[4,5-b]pyridin-3-yl group.

A quinolin-2-yl benzoic acid may be produced by the process of the present invention in the following manner. First, a 2-carboxyphenyl-4-quinolinecarboxylic acid represented by the formula (1) is heated in an appropriate solvent. In this step, a decarboxylation accelerator such as an appropriate acid or base may be used together with the solvent. However, it is preferable to use no decarboxylation accelerator or to use an acid. The heating temperature preferably ranges from 100 to 250 °C, more preferably from 130 to 200 °C. The reaction time ranges from 0.1 to 10 hours, preferably from 0.5 to 2 hours.

It is preferable to use a solvent having a boiling point of from 100 to 250 °C, more preferably from 150 to 230 °C. Suitable examples of the solvent include diphenyl ether, N,N-dimethylacteamide, quinoline, o-dichlorobenzene and xylene. It is preferable to use from 10 to 10,000 ml of the solvent per mole of the 2-carboxyphenyl-4-quinolinecarboxylic acid.

The acid to be used as the decarboxylation accelerator may be selected from among organic acids, mineral acids and Lewis acids, which are highly stable to heat. Suitable examples of the acid include benzenesulfonic acid, p-toluenesulfonic acid, camphorsulfonic acid, hydrochloric acid, sulfuric acid, boric acid, copper oxide, boron trifluoride and acidic alumina. It is particularly preferable to use conc. sulfuric acid.

The base to be used as the decarboxylation accelerator may be selected from among organic amines, organic acid salts and alkalis. Suitable examples thereof include triethylamine, diisopropylamine, N,N-dimethylaniline, sodium acetate, potassium acetate, sodium methoxide, sodium ethoxide, sodium phenoxide, sodium hydroxide, potassium hydroxide, calcium hydroxide, magnesium hydroxide, sodium carbonate, potassium carbonate, sodium hydrogencarbonate, calcium carbonate and basic alumina.

The decarboxylation accelerator is used in an amount of preferably from 0.0001 to 100 mols, more preferably 0.001 to 10 mols, per mol of the 2-carboxyphenyl-4-quinolinecarboxylic acid.

After allowing to cool to room temperature, the reaction mixture thus obtained is poured into water cooled with ice. Then the mixture is made alkaline by adding an appropriate base such as sodium carbonate or sodium hydroxide. The aqueous layer is washed with an appropriate organic solvent such as dichloromethane or toluene and acidified with diluted hydrochloric acid. The precipitate thus formed is collected by filtration or extraction with an appropriate organic solvent such as dichloromethane or ethyl acetate. Thus the quinolin-2-yl benzoic acid represented by the formula (2) can be obtained.

The 2-carboxyphenyl-4-quinolinecarboxylic acid to be used as the starting material in the process of the present invention can be easily synthesized from an isatin and an acylbenzoic acid which are both commercially available or can be easily synthesized. The synthesis may be performed in accordance with, for example, the method described in JP-A-6-16641. A compound wherein R² is -CH₂Q can be obtained by converting the methyl group of a compound, wherein R² is a methyl group, into -CH₂L, wherein L represents a leaving group, and then reacting this L with the above-mentioned organic compound having an -NH- group. This process for the formation of -CH₂Q can be performed in accordance with the methods described in JP-A-6-16659 and JP-A-6-80664 as cited above.

Alternatively, a compound wherein R² is -CH₂Q may be produced by reacting an isatin represented by the following formula (5) with an acylbenzoic acid represented by the following formula (6). Thus a compound represented by the formula (1) wherein R² is -CH₂Q can be obtained. 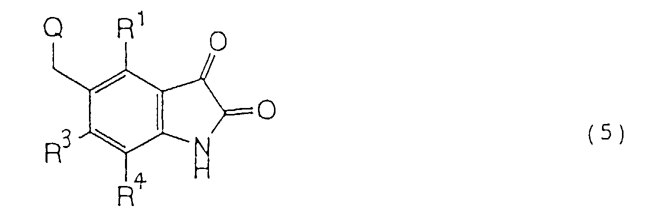 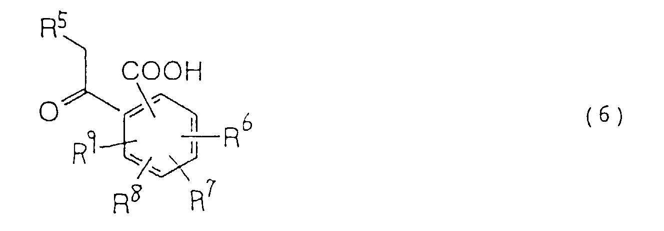 wherein R¹, R³ through R⁹ and Q are defined as above.

This reaction can be carried out by reacting the isatin compounds with the acyl benzoic acid compounds in a reaction medium in the presence of a base. For example, both compounds are allowed to react in a basic aqueous solution under heating. As a base, alkaline metal hydroxides such as sodium hydroxide or potassium hydroxide are preferred. A preferable reaction medium is water. The acylbenzoic acid compounds are preferably used in an amount of 0.5 to 10 mols, particularly 0.5 to 2 mols, per mol of the isatin compounds. The base is preferably used in an amount of 1 to 4 mols, particularly 2 to 3 mols, per mol of the acylbenzoic acid compounds. The reaction can be carried out at a temperature of from 50°C to the reflux temperature of the reaction medium, particularly 80°C to the reflux temperature, for 0.5 to 10 hours, suitably 1 to 3 hours.

The purification methods after the decarboxylation reaction are not particularly limited, but the following method is preferably used. After allowing to cool to room temperature, the reaction mixture was poured into water cooled with ice, the aqueous layer was washed with an organic solvent such as ether and acidified with diluted hydrochloric acid to form a precipitate, and the precipitate was collected by filtration to obtain the desired product.

The isatin of the formula (5) serving as the starting material in this process can be produced by, for example, the following method.

The isatin represented by the formula (5) can be obtained by reacting a substituted aniline compound represented by the following formula (7) with chloral hydrate and a hydroxylamine hydrochloride in an acidic aqueous solution to thereby form isonitrosoacetanilide followed by the cyclization by the Sandmeyer's method. 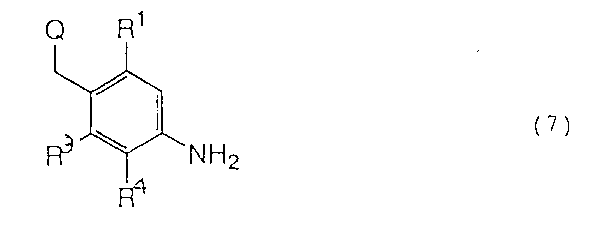 wherein R¹, R³, R⁴ and Q are defined as above.

The substituted aniline compound represented by the formula (7) can be obtained by reacting a substituted nitro compound represented by the following formula (8) in a hydrogen atmosphere in the presence of a catalytic hydrogenation catalyst (palladium, Raney nickel, etc.). Alternatively, the same reaction can be performed by using a metallic hydrogenation agent instead of the above-mentioned catalytic hydrogenation catalyst. 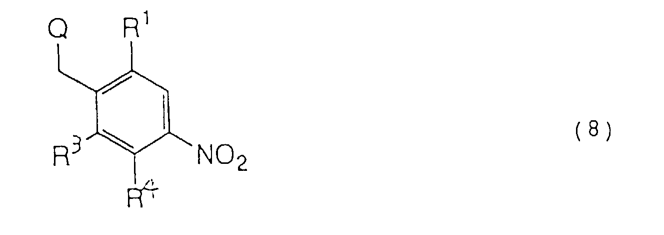 wherein R¹, R³, R⁴ and Q are defined as above.

The substituted nitro compound represented by the formula (8) can be obtained by reacting the above-mentioned organic compound having an -NH- group represented by Q-H with a compound represented by the following formula (9) in the presence of a base, such as sodium hydride, sodium carbonate, potassium carbonate or sodium methoxide, in a nonprotic solvent, such as N,N-dimethylformamide, N,N-dimethylacetamide, tetrahydrofuran or dioxane, at a temperature of from 0 °C to the reflux temperature of the solvent. In the formula (9), L represents a leaving group such as a halogen atom (for example, chlorine, bromine, iodine) or a methanesulfonyloxy or p-toluenesulfonyloxy group. 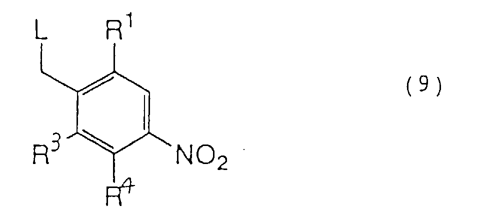 wherein R¹, R³ and R⁴ are defined as above.

To further illustrate the present invention in greater detail, and not by way of limitation, the following Examples will be given.

### Reference Example 1

In 15 ml of water were dissolved 14.7 g (0.1 mol) of isatin and 8 g (0.2 mol) of sodium hydroxide. Then, 16.4 g (0.1 mol) of 2-acetylbenzoic acid was added thereto and the resulting mixture was heated at 90 °C under reflux. When the reaction became mild, the mixture was heated at 115 °C for additional 1 hour. After allowing to cool to room temperature, it was poured into 300 g of water cooled with ice. The aqueous layer was washed with ether and acidified with 2 M hydrochloric acid. The precipitate thus formed was collected by filtration to thereby give 9.85 g of 2-(2-carboxyphenyl)-4-quinolinecarboxylic acid.
NMR (270 MHz, CD₃COCD₃) δ 7.70 - 8.70 (m,9H).

### Reference Example 2

The procedure of Reference Example 1 was repeated but using 10.0 g (62.1 mmol) of 5-methylisatin instead of the isatin. Thus 6.55 g of 2-(2-carboxyphenyl)-6-methyl-4-quinolinecarboxylic acid was obtained.
NMR (270 MHz, CD₃COCD₃) δ 7.70 - 8.70 (m,8H); 2.66 (s,3H).

### Reference Example 3

To 1,200 ml of an aqueous solution of 90 g of chloral hydrate was added 1,300 g of sodium sulfate decahydrate and subsequently and 300 ml of an aqueous solution containing 54 g of o-methylaniline and 43 ml of conc. hydrochloric acid. After further adding 50 ml of an aqueous solution of 110 g of hydroxyamine hydrochloride, the mixture was heated under reflux for 0.5 hour. Then the reaction mixture was cooled with ice and the crystals thus precipitated were collected by filtration. After air-drying, the mixture was slowly dissolved in 325 ml of conc. sulfuric acid, which had been previously heated to 50 °C, under vigorously stirring at a temperature of 75 °C or below. After the completion of the addition, the mixture was heated at 80 °C for 0.5 hour. After allowing to cool, it was poured onto 3 kg of ice. The crystals thus precipitated were collected by filtration to thereby give 33 g of 7-methylisatin.

The procedure of Reference Example 1 was repeated but using 80.0 g of 7-methylisatin synthesized by the method described above instead of isatin. Thus 14.9 g of 2-(2-carboxyphenyl)-8-methyl-4-quinolinecarboxylic acid was obtained.
NMR (270 MHz, CD₃COCD₃) δ 7.70 - 8.50 (m,8H); 2.45 (s,3H).

### Reference Example 4

The procedure of Reference Example 2 was repeated but using 1.38 g of 3-benzylidenephthalide instead of the 2-acetylbenzoic acid. Thus 1.97 g of 2-(2-carboxyphenyl)-6-methyl-3-phenyl-4-quinolinecarboxylic acid was obtained.
NMR (270 MHz, CD₃COCD₃) δ 7.3 - 8.1 (m,12H); 2.68 (s,3H).

### Reference Example 5

A mixture comprising 57.2 g of tetrachlorophthalic anhydride, 25.0 g of malonic acid and 60 ml of pyridine was stirred at 70 to 75 °C for 1.5 hours. After adding 200 ml of water and 200 ml of conc. hydrochloric acid, the mixture was boiled for 10 minutes. After allowing to cool, the precipitate was collected by filtration and dried. The solid product thus obtained was extracted with 600 ml of hot ether. The extract was concentrated and the residue was recrystallized from diluted ethanol. Thus 25.2 g of 2-acetyl-3,4,5,6-tetrachlorobenzoic acid was obtained.
NMR (270 MHz, CDCl₃) δ 2.03 (s,3H).
IR (KBr) ν (C=O) 1770 cm⁻¹, ν (OH) 3380 cm⁻¹.

The procedure of Reference Example 2 was repeated but using 2.45 g of the 2-acetyl-3,4,5,6-tetrachlorobenzoic acid obtained above instead of the 2-acetylbenzoic acid. Thus 1.9 g of 2-(2-carboxy-3,4,5,6-tetrachlorophenyl)-6-methyl-4-quinolinecarboxylic acid was obtained.
NMR (270 MHz, CD₃COCD₃) δ 7.3 - 8.5 (m,4H); 2.70 (s,3H).

### Reference Example 6

The procedure of Reference Example 5 was repeated but using 52 g of 3,6-dichlorophthalic anhydride instead of the tetrachlorophthalic anhydride. Thus 14.1 g of 2-acetyl-3,6-dichlorobenzoic acid was obtained. The procedure of Reference Example 2 was repeated but using 2.2 g of the 2-acetyl-3,6-dichlorobenzoic acid thus obtained instead of the 2-acetylbenzoic acid. Thus 1.5 g of 2-(2-carboxy-3,6-dichlorophenyl)-6-methyl-4-quinolinecarboxylic acid was obtained.
NMR (270 MHz, CD₃COCD₃) δ 7.5 - 8.5 (m,6H); 2.68 (s,3H).

### Reference Example 7

The procedure of Reference Example 5 was repeated but using 62 g of 4,5-dichlorophthalic anhydride instead of the tetrachlorophthalic anhydride. Thus 19.5 g of 2-acetyl-4,5-dichlorobenzoic acid was obtained. The procedure of Reference Example 2 was repeated but using 2.32 g of the 2-acetyl-4,5-dichlorobenzoic acid thus obtained instead of the 2-acetylbenzoic acid. Thus 1.2 g of 2-(2-carboxy-4,5-dichlorophenyl)-6-methyl-4-quinolinecarboxylic acid was obtained.
NMR (270 MHz, CD₃COCD₃) δ 7.3 - 8.7 (m,6H); 2.67 (s,3H).

### Reference Example 8

The procedure of Reference Example 2 was repeated but using 15.8 g of 4-acetylbenzoic acid instead of the 2-acetylbenzoic acid. Thus 8.2 g of 2-(4-carboxyphenyl)-6-methyl-4-quinolinecarboxylic acid was obtained.
NMR (270 MHz, Cd₃COCD₃) δ 7.70 - 8.70 (m,8H); 2.69 (s,3H).

### Reference Example 9

In 20 ml of methanol was suspended 4.10 g of the 2-(2-carboxyphenyl)-6-methyl-4-quinolinecarboxylic acid obtained in Reference Example 2. Then 1.5 ml of thionyl chloride was added thereto while maintaining the mixture at -10 °C. After stirring over day and night, the mixture was concentrated under reduced pressure. The residue was purified by flash chromatography on silica gel with the use of chloroform to thereby give 2.45 g of methyl 2-(2-methoxycarbonylphenyl)-6-methyl-4-quinolinecarboxylate.
NMR (270 MHs, CDCl₃) 6 7.5 -8.6 (m,8H); 4.0 (s,3H); 3.6 (s,3H); 2.6 (s,3H).

Four ml of carbon tetrachloride was added to 540 mg of the above-mentioned methyl 2-(2-methoxycarbonylphenyl)-6-methyl-4-quinolinecarboxylate. Further, 315 mg of N-bromosuccinimide and 8.7 mg of azobisisobutyronitrile were added thereto and the resulting mixture was heated under reflux for 1 hour. The mixture was concentrated under reduced pressure and the residue was dissolved in dichloromethane and washed with water. The organic layer was dried over magnesium sulfate and filtered. Then the filtrate was concentrated under reduced pressure.

The residue thus obtained was dissolved in 2 ml of N,N-dimethylformamide. After adding 44.6 mg of 2-butyl-4-chloro-1H-imidazole-5-carbaldehyde and 36.3 mg of potassium carbonate, the mixture was stirred over day and night at room temperature. Then it was filtered and the filtrate was concentrated under reduced pressure. The resulting residue was purified by flash chromatography on silica gel with the use of a solvent mixture of hexane/ethyl acetate (5 : 1 to 4 : 1) to thereby give 65.5 mg of methyl 6-(2-butyl-4-chloro-5-formyl-1H-imidazol-1-yl)methyl-2-(2-methoxycarbonylphenyl)-4-quinolinecarboxylate.
NMR (270 MHz, CDCl₃) δ 9.8 (s,1H); 7.5 - 8.5 (m,8H); 5.8 (s,2H); 4.0 (s,3H); 3.6 (s,3H); 2.7 (t,J=8Hz,2H); 1.7 (m,2H); 1.4 (m,2H); 0.8 (t, J=7Hz,3H).

In a solvent mixture comprising 2 ml of tetrahydrofuran and 2 ml of methanol was dissolved 65.5 mg of the methyl 6-(2-butyl-4-chloro-5-formyl-1H-imidazol-1-yl)methyl-2-(2-methoxycarbonylphenyl)-4-quinolinecarboxylate obtained above. After stirring, 5.24 mg of sodium borohydride was added thereto and the mixture was reacted at room temperature for 1 hour. After concentrating under reduced pressure, dichloromethane was added to the resulting residue followed by washing with a saturated aqueous solution of sodium chloride. The organic layer was dried over magnesium sulfate and filtered. Then the filtrate was concentrated under reduced pressure to thereby give 50.6 mg of methyl 6-(2-butyl-4-chloro-5-hydroxymethyl-1H-imidazol-1-yl)methyl-2-(2-methoxycarbonylphenyl)-4-quinolinecarboxylate.
NMR (270 MHz, CDCl₃) δ 7.4 - 8.4 (m,8H); 5.4 (s,2H); 4.5 (bs,2H); 4.0 (s,3H); 3.6 (s,3H); 2.8 (bs,1H); 2.6 (t,J=8Hz,2H); 1.6 (m,2H); 1.3 (m,2H); 0.8 (t,J=7Hz,3H).

To 50.6 mg of the methyl 6-(2-butyl-4-chloro-5-hydroxymethyl-1H-imidazol-1-yl)methyl-2-(2-methoxycarbonylphenyl)-4-quinolinecarboxylate obtained above were added 33.5 mg of sodium hydroxide, 1 ml of water and 0.3 ml of ethanol. The mixture was stirred over day and night at room temperature. After concentrating under reduced pressure, it was divided into an aqueous layer and an ether layer. The aqueous layer was acidified with 1 N hydrochloric acid and extracted with ethyl acetate to thereby give 44.2 mg of 6-(2-butyl-4-chloro-5-hydroxymethyl-1H-imidazol-1-yl)methyl-2-(2-carboxyphenyl)-4-quinolinecarboxylic acid.
NMR (270 MHz, CD₃OD) δ 7.6 - 8.6 (m,8H); 5.6 (s,2H); 4.6 (s,2H); 2.7 (t,J=8Hz,2H); 1.6 (m,2H); 1.4 (m,2H); 0.9 (m,3H).

### Reference Example 10

Four ml of carbon tetrachloride was added to 540 mg of methyl 2-(2-methoxycarbonylphenyl)-6-methyl-4-quinolinecarboxylate obtained by the method described in the first stage of Reference Example 9. Further, 315 mg of N-bromosuccinimide and 8.7 mg of azobisisobutyronitrile were added thereto and the resulting mixture was heated under reflux for 1 hour. The mixture was concentrated under reduced pressure and the residue was dissolved in dichloromethane and washed with water. The organic layer was dried over magnesium sulfate and filtered. Then the filtrate was concentrated under reduced pressure.

The residue thus obtained was dissolved in 2 ml of N,N-dimethylformamide. After adding 52 mg of 2-ethyl-5,7-dimethyl-1H-imidazo[4,5-b]pyridine and 8.3 mg of sodium hydride, the mixture was stirred over day and night at room temperature. Then it was filtered and the filtrate was concentrated under reduced pressure. The resulting residue was purified by flash chromatography on silica gel with the use of a solvent mixture of hexane/ethyl acetate (5 : 1 to 4 : 1) to thereby give 44 mg of methyl 6-(2-ethyl-5,7-dimethyl-3H-imidazo[4,5-b]pyridin-3-yl)methyl-2-(2-methoxycarbonylphenyl)-4-quinolinecarboxylate.
NMR (270 MHz, CDCl₃) δ 7.5 - 8.5 (m,8H); 7.0 (s,1H); 5.8 (s,2H); 4.0 (s,3H); 3.6 (s,3H); 2.7 (q,J=8Hz,2H); 2.7 (s,3H); 2.6 (s,3H); 1.7 (t,J=8Hz,3H).

The procedure of Reference Example 9 was repeated but using 44 mg of the methyl 6-(2-ethyl-5,7-dimethyl-3H-imidazo[4,5-b]pyridin-3-yl)methyl-2-(2-methoxycarbonylphenyl)-4-quinolinecarboxylate obtained above instead of the methyl 6-(2-butyl-4-chloro-5-hydroxymethyl-1H-imidazol-1-yl)methyl-2-(2-methoxycarbonylphenyl)-4-quinolinecarboxylate to thereby give 18 mg of 6-(2-ethyl-5,7-dimethyl-3H-imidazo[4,5-b]pyridin-3-yl)methyl-2-(2-carbonylphenyl)-4-quinolinecarboxylic acid.
NMR (270 MHz, CDCl₃) 6 7.5 - 8.5 (m,8H); 7.0 (s,1H); 5.7 (s,2H); 2.8 (q,J=8Hz,2H); 2.7 (s,3H); 2.6 (s,3H); 1.7 (t,J=8Hz,3H).

### Example 1

Ten g of the 2-(2-carboxyphenyl)-4-quinolinecarboxylic acid obtained in Reference Example 1 was dissolved in 200 ml of quinoline. After adding 5 ml of conc. sulfuric acid, the mixture was heated at 160 °C for 1 hour. Then the reaction mixture was poured into water, made alkaline by adding sodium carbonate and washed with toluene. The aqueous layer was neutralized with 3 M hydrochloric acid until a precipitate was formed. Then the precipitate was collected by filtration to thereby give 5.1 g of 2-(quinolin-2-yl)benzoic acid.
NMR (270 MHz, CD₃OD) δ 7.58 - 8.32 (m,10H).

### Example 2

In 1 ml of N,N-dimethylacetamide was dissolved 0.2 g of the 2-(2-carboxyphenyl)-6-methyl-4-quinolinecarboxylic acid obtained in Reference Example 2 and heated at 180 °C for 1 hour. Then the reaction mixture was poured into water, made alkaline by adding sodium carbonate and washed with dichloromethane. The aqueous layer was neutralized with 3 M hydrochloric acid until a precipitate was formed. After extracting with dichloromethane, 60 mg of 2-(6-methylquinolin-2-yl)benzoic acid was obtained.
NMR (270 MHz, CD₃OD) δ 7.58 - 8.32 (m,9H); 2.58 (s,3H).

### Example 3

Five g of the 2-(2-carboxyphenyl)-6-methyl-4-quinolinecarboxylic acid obtained in Reference Example 2 was dissolved in 10 ml of quinoline. After adding 20 mg of conc. sulfuric acid, the mixture was heated at 160 °C for 1 hour. Then the reaction mixture was poured into water, made alkaline by adding sodium carbonate and washed with toluene. The aqueous layer was neutralized with 3 M hydrochloric acid until a precipitate was formed and the precipitate was collected by filtration to thereby give 3.94 g of 2-(6-methylquinolin-2-yl)benzoic acid.
NMR (270 MHz, CD₃OD) δ 7.58 - 8.32 (m,9H); 2.58 (s,3H).

### Example 4

To 1 ml of diphenyl ether was added 0.2 g of the 2-(2-carboxyphenyl)-6-methyl-4-quinolinecarboxylic acid obtained in Reference Example 2. After adding 123 mg of p-toluenesulfonic acid, the mixture was heated at 160 °C for 1 hour. Then the reaction mixture was poured into water, made alkaline by adding sodium hydroxide and washed with ethyl acetate. The aqueous layer was neutralized with 3 M hydrochloric acid until a precipitate was formed. After extracting with ethyl acetate, 80 mg of 2-(6-methylquinolin-2-yl)benzoic acid was obtained.
NMR (270 MHz, CD₃OD) δ 7.58 - 8.32 (m,9H); 2.58 (s,3H).

### Example 5

To 3 ml of diphenyl ether was added 0.5 g of the 2-(2-carboxyphenyl)-6-methyl-4-quinolinecarboxylic acid obtained in Reference Example 2. After adding 6.5 mg of copper oxide, the mixture was heated at 170 °C for 1 hour. Then the reaction mixture was poured into water, made alkaline by adding sodium hydroxide and washed with ethyl acetate. The aqueous layer was neutralized with 3 M hydrochloric acid until a precipitate was formed. After extracting with ethyl acetate, 0.2 g of 2-(6-methylquinolin-2-yl)benzoic acid was obtained.
NMR (270 MHz, CD₃OD) δ 7.58 - 8.32 (m,9H); 2.58 (s,3H).

### Example 6

In 5 ml of quinoline was dissolved 0.1 g of the 2-(2-carboxyphenyl)-8-methyl-4-quinolinecarboxylic acid obtained in Reference Example 3 and heated at 170 °C for 1.5 hours. Then the reaction mixture was poured into water, made alkaline by adding sodium carbonate and washed with toluene. The aqueous layer was neutralized with 3 M hydrochloric acid until a precipitate was formed. After extracting with dichloromethane, 0.05 g of 2-(8-methylquinolin-2-yl)benzoic acid was obtained.
NMR (270 MHz, CD₃OD) δ 7.5 - 8.3 (m,9H); 2.63 (s,3H).

### Example 7

One g of the 2-(2-carboxyphenyl)-6-methyl-3-phenyl-4-quinolinecarboxylic acid obtained in Reference Example 4 was dissolved in 20 ml of quinoline. After adding 200 mg of conc. sulfuric acid, the mixture was heated at 170 °C for 1 hour. Then the reaction mixture was poured into water, made alkaline by adding sodium carbonate and washed with toluene. The aqueous layer was neutralized with 3 M hydrochloric acid until a precipitate was formed and the precipitate was collected by filtration to thereby give 0.6 g of 2-(6-methyl-3-phenylquinolin-2-yl)benzoic acid.
NMR (270 MHz, CD₃OD) δ 7.5 - 8.3 (m,13H); 2.57 (s,3H).

### Example 8

One g of the 2-(2-carboxy-3,4,5,6-tetrachlorophenyl)-6-methyl-4-quinolinecarboxylic acid obtained in Reference Example 5 was dissolved in 15 ml of quinoline. After adding 200 mg of conc. sulfuric acid, the mixture was heated at 160 °C for 1 hour. Then the reaction mixture was poured into water, made alkaline by adding sodium carbonate and washed with toluene. The aqueous layer was neutralized with 3 M hydrochloric acid until a precipitate was formed and the precipitate was collected by filtration to thereby give 0.6 g of 2-(6-methylquinolin-2-yl)-3,4,5,6-tetrachlorobenzoic acid.
NMR (270 MHz, CD₃OD) δ 7.3 - 8.3 (m,5H); 2.61 (s,3H).

### Example 9

One g of the 2-(2-carboxy-3,6-dichlorophenyl)-6-methyl-4-quinolinecarboxylic acid obtained in Reference Example 6 was dissolved in 15 ml of quinoline. After adding 250 mg of conc. sulfuric acid, the mixture was heated at 160 °C for 1 hour. Then the reaction mixture was poured into water, made alkaline by adding sodium carbonate and washed with toluene. The aqueous layer was neutralized with 3 M hydrochloric acid until a precipitate was formed and the precipitate was collected by filtration to thereby give 0.55 g of 2-(6-methylquinolin-2-yl)-3,6-dichlorobenzoic acid.
NMR (270 MHz, CD₃OD) δ 7.3 - 8.3 (m,7H); 2.60 (s,3H).

### Example 10

One g of the 2-(2-carboxy-4,5-dichlorophenyl)-6-methyl-4-quinolinecarboxylic acid obtained in Reference Example 7 was dissolved in 15 ml of quinoline. After adding 250 mg of conc. sulfuric acid, the mixture was heated at 160 °C for 1.5 hours. Then the reaction mixture was poured into water, made alkaline by adding sodium carbonate and washed with toluene. The aqueous layer was neutralized with 3 M hydrochloric acid until a precipitate was formed and the precipitate was collected by filtration to thereby give 0.57 g of 2-(6-methylquinolin-2-yl)-4,5-dichlorobenzoic acid.
NMR (270 MHz, CD₃OD) δ 7.3 - 8.3 (m,7H); 2.58 (s,3H).

### Example 11

In 5 ml of quinoline was dissolved 0.1 g of the 2-(4-carboxyphenyl)-6-methyl-4-quinolinecarboxylic acid obtained in Reference Example 8 and heated at 170 °C for 0.5 hour. Then the reaction mixture was poured into water, made alkaline by adding sodium carbonate and washed with toluene. The aqueous layer was neutralized with 3 M hydrochloric acid until a precipitate was formed. After extracting with dichloromethane, 0.06 g of 4-(6-methylquinolin-2-yl)benzoic acid was obtained.
NMR (270 MHz, CD₃OD) δ 7.5 - 8.3 (m,9H); 2.56 (s,3H).

### Example 12

In 10 ml of quinoline was dissolved 44.2 mg of the 6-(2-butyl-4-chloro-5-hydroxymethyl-1H-imidazol-1-yl)methyl-2-(2-carboxyphenyl)-4-quinolinecarboxylic acid obtained in Reference Example 9. After adding 100 mg of conc. sulfuric acid, the mixture was heated at 170 °C for 1 hour. Then the reaction mixture was poured into water, made alkaline by adding sodium carbonate and washed with toluene. The aqueous layer was neutralized with 3 M hydrochloric acid until a precipitate was formed. Then the precipitate was collected by filtration and purified by flash chromatography on silica gel with the use of chloroform/methanol. Thus 25 mg of 2-[6-(2-butyl-4-chloro-5-hydroxymethyl-1H-imidazol-1-yl)methylquinolin-2-yl]benzoic acid was obtained.
NMR (270 MHz, CDCl₃) δ 7.2 - 8.2 (m,9H); 5.4 (s,2H); 4.5 (s,2H); 2.6 (t,J=8Hz,2H); 1.2 - 1.8 (m,4H); 1.4 (m,2H); 0.8 (t,J=8Hz,3H).

### Example 13

Eighteen mg of the 6-(2-ethyl-5,7-dimethyl-3H-imidazo[4,5-b]pyridin-3-yl)methyl-2-(2-carboxyphenyl)-4-quinolinecarboxylic acid obtained in Reference Example 10 was dissolved in 5 ml of quinoline. After adding 0.5 ml of conc. sulfuric acid, the mixture was heated at 170 °C for 1 hour. Then the reaction mixture was poured into water, made alkaline by adding sodium carbonate and washed with toluene. The aqueous layer was neutralized with 3 M hydrochloric acid until a precipitate was formed. Then the precipitate was collected by filtration and purified by flash chromatography on silica gel with the use of chloroform/methanol. Thus 12 mg of 2-[6-(2-ethyl-5,7-dimethyl-3H-imidazo[4,5-b]pyridin-3-yl)methylquinolin-2-yl]benzoic acid was obtained.
NMR (270 MHz, CDCl₃) δ 7.5 - 8.2 (m,8H); 7.4 (s,1H); 7.0 (s,1H); 5.7 (s,2H); 2.8 (q,J=8Hz,2H); 2.7 (s,3H); 2.6 (s,3H); 1.7 (t,J=8Hz,3H).

### Reference Example 11

### Synthesis of 2-propanoylbenzoic acid:

Twenty g (136 mmol) of phthalic anhydride, 35 ml (270 mmol) of propionic anhydride and 2.6 g (28 mmol) of sodium propionate were heated at 80 °C under stirring for 45 minutes. After allowing to cool, the reaction mixture was poured into a 10 % aqueous solution of acetic acid. The solid matter thus precipitated was collected by filtration and recrystallized from ethanol. The solid product thus obtained was dissolved in 20 ml of a 1.5 N aqueous solution of sodium hydroxide and heated under reflux for 3 hours. After allowing to cool, the reaction mixture was acidified with 20 % hydrochloric acid and extracted with methylene chloride. The organic layer was dried over magnesium sulfate, filtered and concentrated under reduced pressure to thereby give 6.4 g (36 mmol) of 2-propanoylbenzoic acid.
NMR (270 MHz, CDCl₃) δ 7.5 - 8.0 (m,4H); 3.95 (q,J=9.3Hz,2H); 1.38 (t,J=9.3Hz,3H).

### Reference Example 12

### Synthesis of 3-[(4-nitrophenyl)methyl]-2-butyl-3H-imidazo[4, 5-b]pyridine:

In 550 ml of N,N-dimethylformamide was dissolved 18.3 g (104 mmol) of 2-butyl-1H-imidazo[4,5-b]pyridine and the resulting mixture was added to a 60 % aqueous solution of 4.58 g (115 mmol) of sodium hydroxide. After stirring for 30 minutes, 25 g (116 mmol) of p-nitrobenzyl bromide was added to the solution and the resulting mixture was stirred at room temperature for 16 hours. After concentrating under reduced pressure, the residue was dissolved in 300 ml of ethyl acetate. The organic layer was washed with water, dried over magnesium sulfate and filtered. After concentrating under reduced pressure, the residue was purified by silica gel column chromatography with the use of a solvent mixture of toluene/ethyl acetate (2 : 1) to thereby give 19.1 g of 3-[(4-nitrophenyl)-methyl]-2-butyl-3H-imidazo[4,5-b]pyridine.
NMR (270 MHz, CDCl₃) δ 8.33 (dd,J=8.3Hz,1H); 8.17 (d,J=8.4 Hz,2H); 8.04 (d,J=8.1Hz,1H); 7.22 - 7.31 (m,3H); 5.59 (s,2H); 2.79 (t,J=7.3Hz,2H); 1.75 - 1.86 (m,2H); 1.34 - 1.48 (m,2H); 0.92 (t,J=7.3 Hz,3H).

### Reference Example 13

### Synthesis of 3-[(4-aminophenyl)methyl]-2-butyl-3H-imidazo[4,5-b]pyridine:

In 250 ml of ethanol was dissolved 19.1 g (61.7 mmol) of3-[(4-nitrophenyl)methyl]-2-butyl-3H-imidazo[4,5-b]pyridine obtained by the method described in Reference Example 12. After adding 1 g of active carbon carrying 5 % of palladium, the mixture was stirred in a hydrogen gas atmosphere for 18 hours. After filtering off the insoluble matters through Celite and washing with 100 ml of ethanol, the filtrate was concentrated under reduced pressure to thereby give 15.7 g of 3-[(4-aminophenyl)methyl]-2-butyl-3H-imidazo[4,5-b]pyridine.
NMR (270 MHz, CDCl₃) δ 8.33 (dd,J=1.35,4.86Hz,1H); 7.98 (dd,J=1.35,7.83Hz,1H); 7.2 (dd,J=4.86,7.83Hz,1H); 6.97 (d,J=8.4Hz,2H); 6.58 (d,J=8.4Hz,2H); 5.37 (s,2H); 3.6 (bs,NH₂); 2.8 (t,J=7.3Hz,2H); 1.72 - 1.84 (m,2H); 1.33 -1.47 (m,2H); 0.92 (t,J=7.3Hz,3H).

### Reference Example 14

### Synthesis of 5-[(2-butyl-3H-imidazo[4,5-b]pyridin-3-yl)methyl]isatin:

Ten g (60.6 mmol) of chloral hydrate was dissolved in 135 ml of water and 135 g of sodium sulfate was added thereto. After further adding 40 ml of a solution of 15.7 g (56.1 mmol) of3-[(4-aminophenyl)methyl]-2-butyl-3H-imidazo[4,5-b]pyridine obtained by the method described in Reference Example 13 in 4.4 N hydrochloric acid and 220 ml of an aqueous solution of 12.3 g (177.4 mmol) of hydroxylamine hydrochloride, the resulting mixture was stirred under reflux for 30 minutes. After allowing to cool to room temperature, the water was removed by decantation and the residue was washed with 150 ml of water cooled with ice. Then it was dissolved in 170 ml of a 1.5 N solution of sodium hydroxide and neutralized with 2 N hydrochloric acid. After filtering, the filtrate was acidified with 2 N hydrochloric acid. The precipitate was collected by filtration, washed with water cooled with ice and dried.

The isonitrosoacetanilide thus obtained was added to 30 ml of conc. sulfuric acid at 50 °C for 40 minutes and then heated at 90 °C for 15 minutes. After cooling to room temperature, it was poured into 200 ml of water cooled with ice. The precipitate was collected by filtration and the residue was dried and purified by silica gel column chromatography with the use of ethyl acetate as a solvent to thereby give 8.4 g of 5-[(2-butyl-3H-imidazo[4,5-b]pyridin-3-yl)methyl]isatin.
NMR (270 MHz, CD₃OD) δ 8.45 (dd,J=1.35,4.86Hz,1H); 8.05 (dd,J=1.35,8.1Hz,1H); 7.51 (d,J=1.9Hz,1H); 7.42 (dd,J= 4.9,8.1Hz,1H); 7.28 (dd,J=1.9,8.6Hz,1H); 6.76 (d,J=8.6 Hz,1H); 5.5 (s,2H); 3.0 (t,J=7.8Hz,2H); 1.66 - 1.79 (m, 2H); 1.35 - 1.49 (m,2H); 0.93 (t,J=7.8Hz,3H).

### Reference Example 15

### Synthesis of 2-(2-carboxyphenyl)-3-methyl-6-[(2-butyl-3H-imidazo[4,5-b]pyridin-3-yl)methyl]-4-quinolinecarboxylic acid:

In 110 ml of a 1 N aqueous solution of sodium hydroxide was dissolved 6.4 g (36 mmol) of 2-propanoylbenzoic acid obtained by the method described in Reference Example 11. Then 9.4 g (36 mmol) of 5-(2-butyl-3H-imidazo[4,5-b]pyridin-3-yl)methylisatin obtained by the method described in Reference Example 14 was added thereto and the mixture was heated under reflux for 4 hours. After adding 100 ml of water and washing with 100 ml of methylene chloride, the aqueous layer was acidified with 2 N hydrochloric acid under stirring and icecooling. The precipitate was collected by filtration and dried to thereby give 14.2 g of 2-(2-carboxyphenyl)-3-methyl-6-[(2-butyl-3H-imidazo[4,5-b]pyridin-3-yl)methyl]-4-quinolinecarboxylic acid.
NMR (270 MHz, CD₃COCD₃) 6 9.2 (m,1H); 8.2 - 8.7 (m,3H); 7.83 (d,J=7.8Hz,1H); 7.4 - 7.9 (m,5H); 5.74 (s,2H); 3.5 (s,3H); 2.88 (t,J=7.8Hz,2H); 2.64 (s,3H); 1.81 - 1.89 (m,2H); 1.38 - 1.45 (m,2H); 0.9 (t, J=7.3Hz,3H).

### Reference Example 16

### Synthesis of 3-[(4-nitrophenyl)methyl]-5,7-dimethyl-2-ethyl-3H-imidazo[4,5-b]pyridine:

The procedure of Reference Example 12 was repeated but using 200 mg of 5,7-dimethyl-2-ethyl-1H-imidazo[4,5-b]pyridine instead of the 2-butyl-1H-imidazo[4,5-b]pyridine. Thus 205 mg of 3-[(4-nitrophenyl)methyl]-5,7-dimethyl-2-ethyl-3H-imidazo[4,5-b]pyridine was obtained.
NMR (270 MHz, CDCl₃) δ 7.2 - 8.3 (m,5H); 5.6 (s,2H); 2.8 (q,J=8Hz,2H); 2.7 (s,3H); 2.6 (s,3H); 1.3 (t,J=8Hz,3H).

### Reference Example 17

### Synthesis of 3-[(4-aminophenyl)methyl]-5,7-dimethyl-2-ethyl-3H-imidazo[4,5-b]pyridine:

Starting from 202 mg of 3-[(4-nitrophenyl)methyl]-5,7-dimethyl-2-ethyl-3H-imidazo[4,5-b]pyridine obtained by the method described in Reference Example 16 , the procedure of Reference Example 3 was repeated to thereby give 162 mg of 3-[(4-aminophenyl)methyl]-5,7-dimethyl-2-ethyl-3H-imidazo[4,5-b]pyridine.
NMR (270 MHz, CDCl₃) δ 7.0 - 8.3 (m,4H); 6.6 (s,1H); 5.4 (s,2H); 3.6 (bs,2H); 2.8 (q,J=8Hz,2H); 2.7 (s,3H); 2.6 (s,3H); 1.3 (t,J=8Hz,3H).

### Reference Example 18

### Synthesis of 5-[(5,7-dimethyl-2-ethyl-3H-imidazo[4,5-b]pyridin-3-yl)methyl]isatin:

Starting from 160 mg of 3-[(4-aminophenyl)methyl]-5,7-dimethyl-2-ethyl-3H-imidazo[4,5-b]pyridine obtained by the method described in Reference Example 17, the procedure of Reference Example 4 was repeated to thereby give 84 mg of 5-[(5,7-dimethyl-2-ethyl-3H-imidazo[4,5-b]pyridin-3-yl)methyl]isatin.
NMR (270 MHz, CD₃OD) δ 7.3 - 8.5 (m,4H); 6.8 (s,1H); 5.5 (s,2H); 2.9 (q,J=8Hz,2H); 2.7 (s,3H); 2.6 (s,3H); 1.5 (t,J=8Hz,3H).

### Reference Example 19

### Synthesis of 2-(2-carboxyphenyl)-6-[(5,7-dimethyl-2-ethyl-3H-imidazo[4,5-b]pyridin-3-yl)methyl]-4-quinolinecarboxylicacid:

By using acetylbenzoic acid and 19 mg of the 5-[(5,7-dimethyl-2-ethyl-3H-imidazo[4,5-b]pyridin-3-yl)methyl]isatin obtained in Reference Example 18, the procedure of Example 1 was repeated to thereby give 27 mg of 2-(2-carboxyphenyl)-6-[(5,7-dimethyl-2-ethyl-3H-imidazo[4,5-b]pyridin-3-yl)methyl]-4-quinolinecarboxylic acid.
NMR (270 MHz, CDCl₃) δ 7.4 - 8.2 (m,8H); 7.0 (s,1H); 5.7 (s,2H); 2.8 (q,J=8Hz,2H); 2.7 (s,3H); 2.6 (s,3H); 1.7 (t,J=8Hz,3H).

## Claims

1. A process for producing a quinolin-2-yl benzoic acid compound characterized by decarboxylating a 2-carboxyphenyl-4-quinolinecarboxylic acid compound in which two carboxyl groups are not substituted and a benzene ring and a quinoline ring may have one or more substituents inert to said decarboxylation reaction.

2. A process as claimed in Claim 1, wherein said decarboxylation is performed in an inert solvent in the presence of an acid at a temperature of 100 to 250 °C.

3. A process as claimed in Claim 1, wherein said 2-carboxyphenyl-4-quinolinecarboxylic acid is a compound represented by the formula (1) and said quinolin-2-yl benzoic acid is a compound represented by the formula (2): 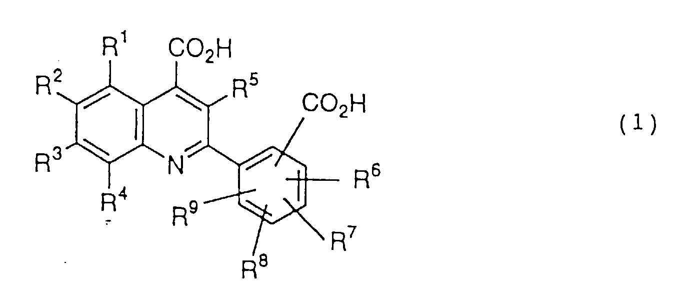 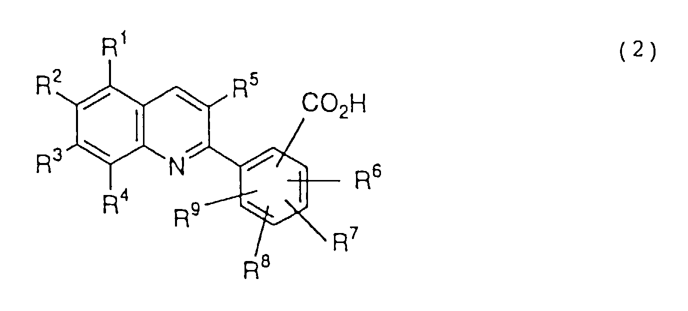 wherein R¹, R³, R⁴, R⁵, R⁶, R⁷, R⁸ and R⁹ each independently represents a hydrogen atom, a halogen atom, an alkyl group having from 1 to 6 carbon atoms, a cycloalkyl group having from 3 to 6 carbon atoms, an aryl group, an aralkyl group, an alkoxy group or -CₘF₂ₘ₊₁; R² represents a hydrogen atom, a halogen atom, an alkyl group having from 1 to 6 carbon atoms, a cycloalkyl group having from 3 to 6 carbon atoms, an aryl group, an aralkyl group, an alkoxy group, -CₙF₂ₙ₊₁ or -CH₂Q, wherein Q represents a monovalent organic group derived from an organic compound having an -NH- group by eliminating the hydrogen atom bonded to said nitrogen atom, and m and n each independently represents an integer of from 1 to 6.

4. A process as claimed in Claim 3, wherein R¹, R³, R⁴ and R⁵ each independently represents a hydrogen atom, an alkyl group having from 1 to 6 carbon atoms or an aryl group; R⁶, R⁷, R⁸ and R⁹ each independently represents a hydrogen atom, a halogen atom or an alkyl group having from 1 to 6 carbon atoms; and R² represents a hydrogen atom, an alkyl group having from 1 to 6 carbon atoms, an aryl group or -CH₂Q, wherein Q represents a monovalent organic group derived from a heterocyclic compound having a nitrogen atom, to which a hydrogen atom is bonded, constituting the ring by eliminating the hydrogen atom bonded to said nitrogen atom.

5. A process as claimed in Claim 1 or 3, wherein said 2-carboxyphenyl-4-quinolinecarboxylic acid is 2-(2-carboxyphenyl)-6-methyl-4-quinolinecarboxylic acid and said quinolin-2-yl benzoic acid is 2-(6-methylquinolin-2-yl) benzoic acid.

6. A process as claimed in Claim 1 or 3, wherein said 2-carboxyphenyl-4-quinolinecarboxylic acid is a 2-(2-carboxyphenyl)-6-substituted-4-quinolinecarboxylic acid and said quinolin-2-yl benzoic acid is a 2-(6-substituted-quinolin-2-yl)benzoic acid and the substituent at the 6-position of each compound is -CH₂Q, wherein Q represents a substituted 1H-imidazol-1-yl group or a substituted 3H-imidazo[4,5-b]pyridin-3-yl group.

7. A process for producing a quinolin-2-yl benzoic acid of the following formula (2'): 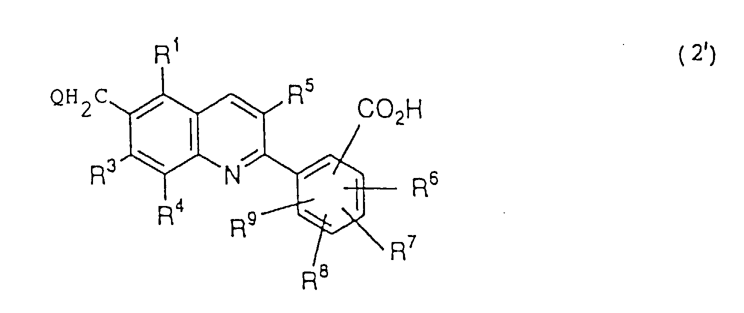 wherein R¹, R³, R⁴, R⁵, R⁶, R⁷, R⁸ and R⁹ each independently represents a hydrogen atom, a halogen atom, an alkyl group having from 1 to 6 carbon atoms, a cycloalkyl group having from 3 to 6 carbon atoms, an aryl group, an aralkyl group, an alkoxy group or -CₘF₂ₘ₊₁; Q represents a monovalent organic group derived from an organic compound having an -NH- group by eliminating the hydrogen atom bonded to said nitrogen atom, and m represents an integer of
from 1 to 6,
comprising the following steps
a) reacting an isatin represented by the formula (5): 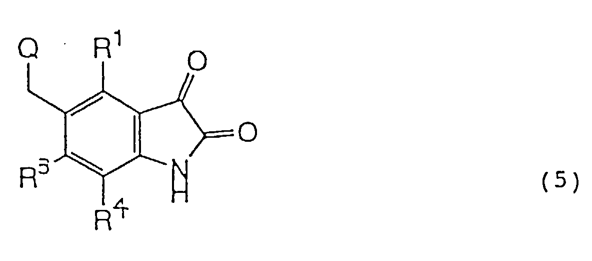 wherein R¹, R³, R⁴ and Q are defined as above, with an acylbenzoic acid represented by the formula (6): 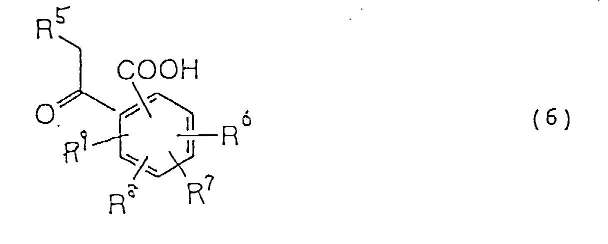 wherein R⁵, R⁶, R⁷, R⁸ and R⁹ are defined as above,to produce a 2-carboxyphenyl-4-quinolinecarboxylic acid represented by the formula (1'): 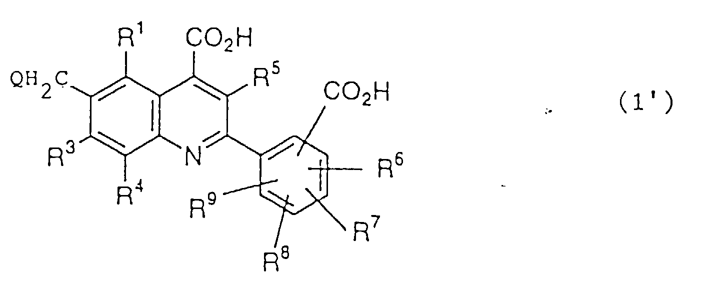 wherein R¹, R³, R⁴, R⁵, R⁶, R⁷, R⁸, R⁹ and Q are defined as above; and
b) decarboxylating the 2-carboxyphenyl-4-quinolinecarboxylic acid of formula (1') to produce the quinolin-2-yl benzoic acid of formula (2').

8. A process as claimed in claim 7, wherein Q is a monovalent organic group derived from a heterocyclic compound having a nitrogen atom, to which a hydrogen atom is bonded, constituting the ring by eliminating the hydrogen atom bonded to said nitrogen atom.

9. A process as claimed in claim 7, wherein Q is a substituted 1H-imidazol-1-yl group or a substituted 3H-imidazo[4,5-b]pyridin-3-yl group.

10. A process as claimed in Claim 7, wherein R¹, R³, R⁴ and R⁵ each independently represents a hydrogen atom, an alkyl group having from 1 to 6 carbon atoms or an aryl group, and R⁶, R⁷, R⁸ and R⁹ each independently represents a hydrogen atom, a halogen atom or an alkyl group having from 1 to 6 carbon atoms.

## Patentansprüche

1. Verfahren zur Herstellung einer Chinolin-2-yl-benzoesäure-Verbindung,
**gekennzeichnet** durch
das Decarboxylieren einer 2-Carboxyphenyl-4-chinolincarbonsäure-Verbindung, in der zwei Carboxy-Gruppen nicht substituiert sind und ein Benzol-Ring und ein Chinolin-Ring einen oder mehrere Substituenten aufweisen können, die gegenüber der Decarboxylierungsreaktion inert sind.

2. Verfahren gemäß Anspruch 1, worin man die Decarboxylierung in einem inerten Lösungsmittel in Gegenwart einer Säure bei einer Temperatur von 100 bis 250°C durchführt.

3. Verfahren gemäß Anspruch 1, worin die 2-Carboxyphenyl-4-chinolincarbonsäure-Verbindung eine durch die Formel (1) dargestellte Verbindung ist und die Chinolin-2-yl-benzoesäure-Verbindung eine durch die Formel (2) dargestellte Verbindung ist: 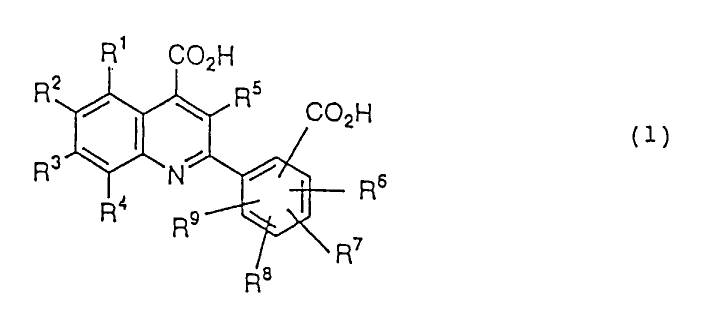 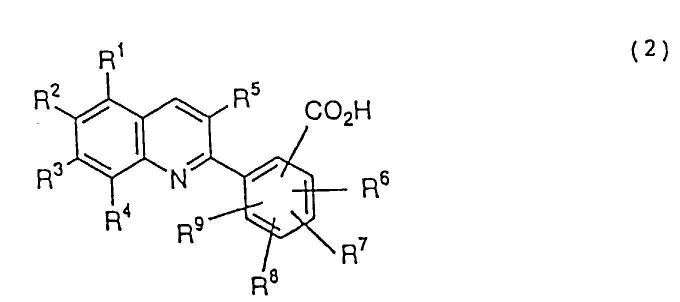 worin R¹, R³, R⁴, R⁵, R⁶, R⁷, R⁸ und R⁹ jeweils unabhängig voneinander ein Wasserstoffatom, ein Halogenatom, eine Alkyl-Gruppe mit 1 bis 6 Kohlenstoffatomen, eine Cycloalkyl-Gruppe mit 3 bis 6 Kohlenstoffatomen, eine Aryl-Gruppe, eine Aralkyl-Gruppe, eine Alkoxy-Gruppe oder -CₘF₂ₘ₊₁ bedeuten; R² ein Wasserstoffatom, ein Halogenatom, eine Alkyl-Gruppe mit 1 bis 6 Kohlenstoffatomen, eine Cycloalkyl-Gruppe mit 3 bis 6 Kohlenstoffatomen, eine Aryl-Gruppe, eine Aralkyl-Gruppe, eine Alkoxy-Gruppe, -CₙF₂ₙ₊₁ oder -CH₂Q bedeutet, worin Q für eine einbindige organische Gruppe steht, die aus einer organischen Verbindung mit einer -NH-Gruppe durch Eliminieren des an das Stickstoffatom gebundenen Wasserstoffatoms abgeleitet wird, und m und n jeweils unabhängig voneinander eine ganze Zahl von 1 bis 6 bedeuten.

4. Verfahren gemäß Anspruch 3, worin R¹, R³, R⁴ und R⁵ jeweils unabhängig voneinander ein Wasserstoffatom, eine Alkyl-Gruppe mit 1 bis 6 Kohlenstoffatomen oder eine Aryl-Gruppe bedeuten; R⁶, R⁷, R⁸ und R⁹ jeweils unabhängig voneinander ein Wasserstoffatom, ein Halogenatom oder eine Alkyl-Gruppe mit 1 bis 6 Kohlenstoffatomen bedeuten; und R² ein Wasserstoffatom, eine Alkyl-Gruppe mit 1 bis 6 Kohlenstoffatomen, eine Aryl-Gruppe oder -CH₂Q bedeutet, worin Q für eine einbindige organische Gruppe steht, die aus einer heterocyclischen Verbindung mit einem Stickstoffatom abgeleitet wird, an das das Wasserstoffatom gebunden ist, unter Eliminieren des an das Stickstoffatom gebundenen Wasserstoffatoms einen Ring ausmachend.

5. Verfahren gemäß Anspruch 1 oder 3, worin die 2-Carboxyphenyl-4-chinolincarbonsäure 2-(2-Carboxyphenyl)-6-methyl-4-chinolincarbonsäure und die Chinolin-2-yl-benzoesäure 2-(6-methylchinolin-2-yl)benzoesäure ist.

6. Verfahren gemäß Anspruch 1 oder 3, worin die Carboxyphenyl-4-chinolincarbonsäure eine 2-(2-Carboxyphenyl)-6-substituierte-4-Chinolincarbonsäure ist und die Chinolin-2-ylbenzoesäure eine 2-(6-substituierte-Chinolin-2-yl)benzoesäure ist und der Substituent in der 6-Position einer jeden Verbindung -CH₂Q ist, worin Q eine substituierte 1H-Imidazol-1-yl-Gruppe oder eine substituierte 3H-Imidazo[4,5-b]pyridin-3-yl-Gruppe ist.

7. Verfahren zur Herstellung einer Chinolin-2-yl-benzoesäure der folgenden Formel (2'): 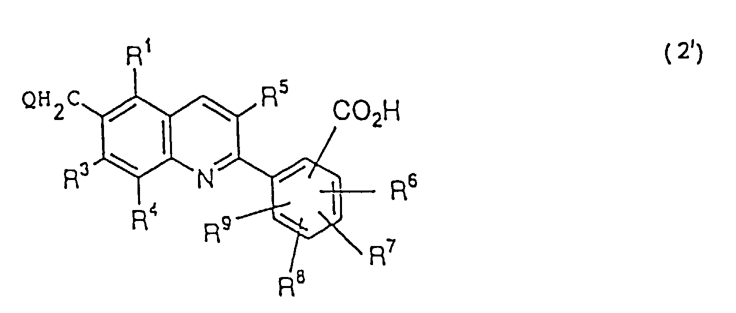 worin R¹, R³, R⁴, R⁵, R⁶, R⁷, R⁸ und R⁹ jeweils unabhängig voneinander ein Wasserstoffatom, ein Halogenatom, eine Alkyl-Gruppe mit 1 bis 6 Kohlenstoffatomen, eine Cycloalkyl-Gruppe mit 3 bis 6 Kohlenstoffatomen, eine Aryl-Gruppe, eine Aralkyl-Gruppe, eine Alkoxy-Gruppe oder -CₘF₂ₘ₊₁ bedeuten; Q für eine einbindige organische Gruppe steht, die aus einer organischen Verbindung mit einer -NH-Gruppe durch Eliminieren des an das Stickstoffatom gebundenen Wasserstoffatoms abgeleitet wird, und m eine ganze Zahl von 1 bis 6 bedeutet,
die folgenden Schritte umfassend:
a) das Umsetzen eines durch die Formel (5) dargestellten Isatins: 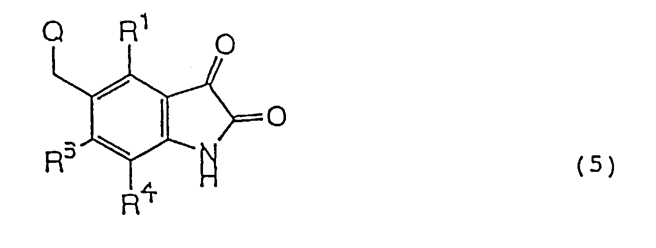 worin R¹, R³, R⁴ und Q wie oben definiert sind, mit einer durch die Formel (6) dargestellten Acylbenzoesäure: 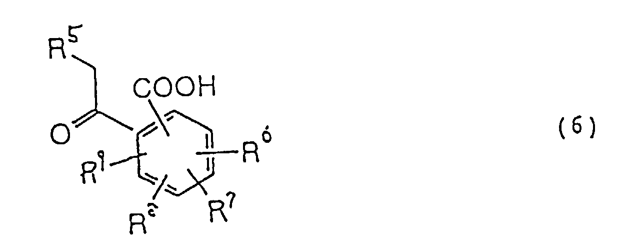 worin R⁵, R⁶, R⁷, R⁸ und R⁹ wie zuvor definiert sind, um eine 2-Carboxyphenyl-4-chinolincarbonsäure der Formel (1') herzustellen: 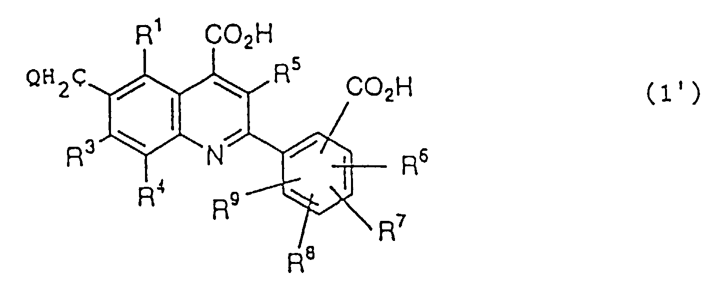 worin R¹, R³, R⁴, R⁵, R⁶, R⁷, R⁸, R⁹ und Q wie zuvor definiert sind; und
b) das Decarboxylieren der 2-Carboxyphenyl-4-chinolincarbonsäure der Formel (1') zur Herstellung der Chinolin-2-yl-benzoesäure der Formel (2').

8. Verfahren gemäß Anspruch 7, worin Q für eine einbindige organische Gruppe steht, die aus einer heterocyclischen Verbindung mit einem Stickstoffatom abgeleitet wird, an das das Wasserstoffatom gebunden ist, unter Eliminieren des an das Stickstoffatom gebundenen Wasserstoffatoms einen Ring ausmachend.

9. Verfahren gemäß Anspruch 7, worin Q eine substituierte 1H-Imidazol-1-yl-Gruppe oder eine substituierte 3H-Imidazo[4,5-b]pyridin-3-yl-Gruppe ist.

10. Verfahren gemäß Anspruch 7, worin R¹, R³, R⁴ und R⁵ jeweils unabhängig voneinander ein Wasserstoffatom, eine Alkyl-Gruppe mit 1 bis 6 Kohlenstoffatomen oder eine Aryl-Gruppe bedeuten, und R⁶, R⁷, R⁸ und R⁸ jeweils unabhängig voneinander ein Wasserstoffatom, ein Halogenatom oder eine Alkyl-Gruppe mit 1 bis 6 Kohlenstoffatomen bedeuten.

## Revendications

1. Procédé pour la préparation d'un dérivé d'acide quinoléin-2-ylbenzoïque caractérisé par la décarboxylation d'un dérivé d'acide 2-carboxyphényl-4-quinoléinecarboxylique, dans lequel les deux groupes carboxyle ne sont pas substitués et le cycle benzénique et le cycle de la quinoléine peuvent comporter un ou plusieurs substituants inertes vis à vis de ladite réaction de décarboxylation.

2. Procédé tel que revendiqué dans la revendication 1, dans lequel ladite décarboxylation est effectuée dans un solvant inerte en présence d'un acide à une température comprise entre 100 et 250°C.

3. Procédé tel que revendiqué dans la revendication 1, dans lequel ledit acide 2-carboxyphényl-4-quinoléinecarboxylique est un composé représenté par la formule (1) et en ce que ledit acide quinoléin-2-ylbenzoïque est un composé représenté par la formule (2) : 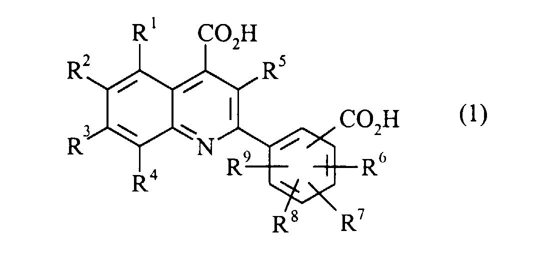 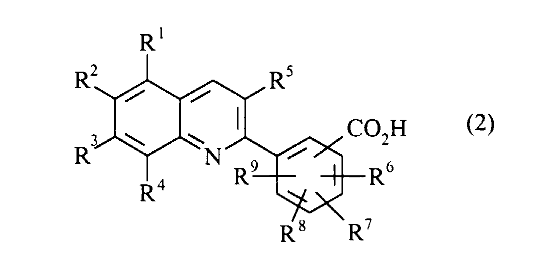 dans lesquelles R¹, R³, R⁴, R⁵, R⁶, R⁷, R⁸ et R⁹ représentent chacun indépendamment un atome d'hydrogène, un atome d'halogène, un groupe alkyle ayant de 1 à 6 atomes de carbone, un groupe cycloalkyle ayant de 3 à 6 atomes de carbone, un groupe aryle, un groupe aralkyle, un groupe alcoxy ou un groupe -CₘF₂ₘ₊₁; R² représente un atome d'hydrogène, un atome d'halogène, un groupe alkyle ayant de 1 à 6 atomes de carbone, un groupe cycloalkyle ayant de 3 à 6 atomes de carbone, un groupe aryle, un groupe aralkyle, un groupe alcoxy, un groupe -CₙF₂ₙ₊₁ ou un groupe -CH₂Q, dans lequel Q représente un groupe organique monovalent dérivé d'un composé organique comportant un groupe -NH- par élimination de l'atome d'hydrogène lié audit atome d'azote et m et n représentent chacun indépendamment un entier compris entre 1 et 6.

4. Procédé tel que revendiqué dans la revendication 3, dans lequel R¹, R³, R⁴ et R⁵ représentent chacun indépendamment un atome d'hydrogène, un groupe alkyle ayant de 1 à 6 atomes de carbone ou un groupe aryle; R⁶, R⁷, R⁸ et R⁹ représentent chacun indépendamment un atome d'hydrogène, un atome d'halogène ou un groupe alkyle ayant de 1 à 6 atomes de carbone; et R² représente un atome d'hydrogène, un groupe alkyle ayant de 1 à 6 atomes de carbone, un groupe aryle ou un groupe -CH₂Q, dans lequel Q représente un groupe organique monovalent dérivé d'un composé hétérocyclique comportant un atome d'azote, auquel est lié un atome d'hydrogène, faisant partie du cycle par élimination de l'atome d'hydrogène lié audit atome d'azote.

5. Procédé tel que revendiqué dans la revendication 1 ou 3, dans lequel ledit acide 2-carboxyphényl-4-quinoléinecarboxylique est l'acide 2-(2-carboxyphényl)-6-méthyl-4-quinoléinecarboxylique et ledit acide quinoléin-2-ylbenzoïque est l'acide 2-(6-méthylquinoléin-2-yl)benzoïque.

6. Procédé tel que revendiqué dans la revendication 1 ou 3, dans lequel ledit acide 2-carboxyphényl-4-quinoléinecarboxylique est l'acide 2-(2-carboxyphényl)-4-quinoléinecarboxylique substitué en position 6 et ledit acide quinoléin-2-ylbenzoïque est l'acide 2-(quinoléin-2-yl)benzoïque substitué en position 6 et le substituant en position 6 pour chaque composé est un groupe -CH₂Q, dans lequel Q représente un groupe 1H-imidazol-1-yle substitué ou un groupe 3H-imidazo[4,5-b]pyridin-3-yle substitué.

7. Procédé pour la préparation d'un acide quinoléin-2-ylbenzoïque de la formule (2') suivante : 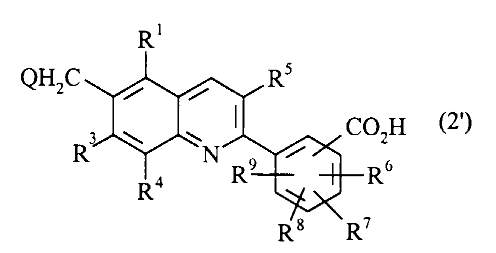 dans laquelle R¹, R³, R⁴, R⁵, R⁶, R⁷, R⁸ et R⁹ représentent chacun indépendamment un atome d'hydrogène, un atome d'halogène, un groupe alkyle ayant de 1 à 6 atomes de carbone, un groupe cycloalkyle ayant de 3 à 6 atomes de carbone, un groupe aryle, un groupe aralkyle, un groupe alcoxy ou un groupe -CₘF₂ₘ₊₁; Q représente un groupe organique monovalent dérivé d'un composé organique comportant un groupe -NH- par élimination de l'atome d'hydrogène lié audit atome d'azote et m représente un entier compris entre 1 et 6, comprenant les étapes suivantes
a) réaction d'un dérivé d'isatine représenté par la formule (5) : 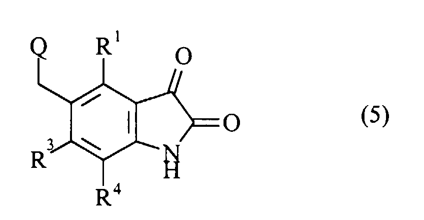 dans laquelle R¹, R³, R⁴ et Q sont tels que définis ci-dessus, avec un acide acylbenzoïque représenté par la formule (6) : 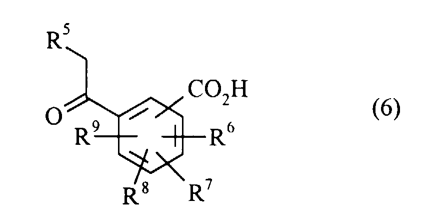 dans laquelle R⁵, R⁶, R⁷, R⁸ et R⁹ sont tels que définis ci-dessus, afin de préparer un acide 2-carboxyphényl-4-quinoléinecarboxylique représenté par la formule (1'): 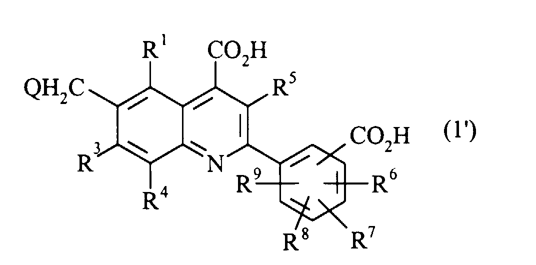 dans laquelle R¹, R³, R⁴, R⁵, R⁶, R⁷, R⁸, R⁹ et Q sont tels que définis ci-dessus; et
b) décarboxylation de l'acide 2-carboxyphényl-4-quinoléinecarboxylique de formule (1') afin de préparer l'acide quinoléin-2-ylbenzoïque de formule (2').

8. Procédé tel que revendiqué dans la revendication 7, dans lequel Q représente un groupe organique monovalent dérivé d'un composé hétérocyclique comportant un atome d'azote, auquel est lié un atome d'hydrogène, faisant partie du cycle par élimination de l'atome d'hydrogène lié audit atome d'azote.

9. Procédé tel que revendiqué dans la revendication 7, dans lequel Q représente un groupe 1H-imidazol-1-yle substitué ou un groupe 3H-imidazo[4,5-b]pyridin-3-yle substitué.

10. Procédé tel que revendiqué dans la revendication 7, dans lequel R', R³, R⁴ et R⁵ représentent chacun indépendamment un atome d'hydrogène, un groupe alkyle ayant de 1 à 6 atomes de carbone ou un groupe aryle et R⁶, R⁷, R⁸ et R⁹ représentent chacun indépendamment un atome d'hydrogène, un atome d'halogène ou un groupe alkyle ayant de 1 à 6 atomes de carbone.
